# EUROPEAN PATENT APPLICATION

(11) **EP 1 754 697 A1**
(43) Date of publication of application: **21.02.2007**
(21) Application number: 05017930.8
(22) Date of filing: 18.08.2005
(51) Int. Cl.: C07C 51/093, C07C 53/21, C07C 53/15

(54) **Preparation of carboxylic acids**

(71) Applicant: Solvay S.A., 1050 Brüssel (BE)
(72) Inventor: Mathieu, Veronique, 1300 Wavre (BE); Buyle, Olivier, 1367 Autre-Eglise (BE); Janssens, Francine, 1800 Vilvoorde (BE)
(74) Representative: Jacques, Philippe

(57) **Abstract**

Carboxylic acids can be prepared by reacting compounds comprising a -CCl₂F group, a CCIF₂ group or a CCl₃ group with sulfur trioxide and sulfuric acid. The reaction is especially suitable for the preparation of 3,3,3-trifluoropropionic acid from CF₃-CH₂-CCl₂F, CF₃-CH₂-CCIF₂, CF₃-CH₂-CCl₃ or a mixture of one or two thereof as starting material. Usually, oleum is used as the source for sulfur trioxide.

In a preferred embodiment, the starting material comprises CF₃-CH₂-CCl₂F and/or CF₃-CH₂-CCIF₂ which is or are prepared in a fluorination reaction between CCl₃-CH₂-CCl₃ and HF. As a source for CCl₃-CH₂-CCl₂, the product of the reaction between CH₂=CCl₂ and CCl₄ is advantageously used.

## Description

The invention relates to the preparation of carboxylic acids by reaction of a starting material comprising compounds with a perchloro or chlorofluorocarbon group and SO₃/H₂SO₄.

Carboxylic acids, especially halogen-substituted carboxylic acids like 3,3,3-trifluoropropionic acid, are intermediates useful in the synthesis of pharmaceuticals and agrochemicals.

Methods for the preparation of such compounds are already known. For the preparation of 3,3,3-trifluoropropionic acid, several different synthetic routes have been described. J. Peters discloses in J. Chem. Eng. Data 16 (1971), page 376ff that 3,3,3-trifluoropropionic acid can be prepared by reacting malonic acid monoethylester with SF₄ and subsequent hydrolysis of the resulting 3,3,3-trifluoropropionic acid ethyl ester with aqueous sulfuric acid. SF₄ is difficult to prepare and to handle. J. Bouillon describes in J. Chem. Soc. Perkin Trans 1, p. 2141 to 2149 the synthesis of trifluoropropionic acid starting with 3-bromopropene and CF₃CdBr / Cul in dimethylformamide and subsequent oxidation. International patent application WO 01/58833 discloses the preparation of trifluoropropionic acid from vinyl acetate which is reacted with trifluoro sulfonylchloride, formation of an intermediate 3,3,3-trifluoropropanal and its oxidation to form the desired acid. H. Yamanaka, T. Takekawa, K. Morita, T. Ishihara and J. T. Gupton disclose in Tetrahedron Letters, 37, p. 1829 to 1832 (1996) a process for the preparation of trifluoropropionic acid whereby t-butyl acetate is reacted with LDA (lithium diisopropylamide) and t-BuMe₂SiCl, then with CF3I in the presence of triethylborane as a catalyst, and subsequent hydrolysis.

Problem of the present invention is to provide a technically feasible process to provide carboxylic acids with 2 to 5 C atoms, especially trifluoropropionic acid.

Thus, subject of the present invention is a process for the preparation of carboxylic acids with the general formula (I) R-COOH wherein R is an alkyl group with 1 to 4 carbon atoms, optionally substituted by 1 or more halogen atoms, by reaction of a starting material of the general formula R-CCl₂F, R- CClF₂ or R-CCl₃ wherein R has the meaning given above, with SO₃ and H₂SO₄ and subsequent hydrolysis, with the exception of the preparation of trifluoroacetic acid from CF₃CCl₃. H₂SO₄ is used in concentrated form.

In principle, the reaction type is suitable for all compounds falling under the general formula given above which do not react with SO₃ and H₂SO₄ in an undesired manner. Preferred are compounds where R is substituted by 1 or more, preferably 2 or more fluorine atoms. Especially preferred are compounds wherein R denotes a group with 2 to 4 carbon atoms. Still more preferred are compounds wherein R is an R'-CH₂ group wherein R' is an alkyl group with 1 to 3 carbon atoms which is substituted by at least 2 fluorine atoms.

The process according to the invention is especially suitable to produce trifluoropropionic acid. As starting compounds, one of CF₃-CH₂-CCl₂F, CF₃-CH₂-CClF₂, CF₃-CH₂-CCl₃ or a mixture of two or more of these compounds is or are very suitable, especially one of CF₃-CH₂-CCl₂F and CF₃-CH₂-CClF₂ or a mixture thereof.

The process of the present invention will now be explained in more detail in view of the preferred embodiment using CF₃-CH₂-CCl₃, CF₃-CH₂-CCl₂F and CF₃-CH₂-CClF₂ or a mixture thereof, especially using CF₃-CH₂-CCl₂F and CF₃-CH₂-CClF₂ or a mixture thereof to produce trifluoropropionic acid.

S03 and H₂SO₄ are preferably entered into the reaction as oleum. The content of SO3 in the oleum is > 0 and may amount up to 50 % by weight in the oleum or even more. A preferred range is 10 to 40 % by weight, more preferably 20 to 40 % by weight. For example, with a content of 20 to 30 % by weight, good results may be obtained.

It is preferred to apply a molar excess of the oleum over the compound or compounds to be hydrolyzed. For example, good results can be obtained when the molar ratio between SO₃ in the oleum and CF₃-CH₂-CCl₂F and CF₃-CH₂-CClF₂ or a mixture thereof is in the range of 1:1 and 15:1, preferably in the range of 1:1 and 10:1, especially in the range of 1:5 and very especially in the range of 1:3.

Advantageously, the reaction is performed at elevated temperature, preferably above 50 °C, especially above 80 °C. Preferably, the reaction temperature is not higher than 160 °C; more preferably it is not higher than 150 °C.

The pressure is not critical. It is preferred to perform the reaction under autogenous pressure.

One advantage of the process of the present invention is that the starting compounds, like oleum, is easily available, and the reaction can be performed in an easy manner. In view of the preferred embodiment, the preparation of trifluoropropionic acid from CF₃-CH₂-CCl₃, CF₃-CH₂-CCl₂F and CF₃-CH₂-CClF₂ or a mixture thereof, and especially from CF₃-CH₂-CCl₂F and CF₃-CH₂-CClF₂ or a mixture thereof, these starting compounds are also very easily obtainable. They can be prepared like described in EP-A-0 522639, e.g. as indicated in the part of said document concerning the state of the art, like described by Henne et al. in J. Am. Chem. Soc. 68 (1946), page 496 by providing 1,1,1-trichloro-1,1,1-trifluoropropane and fluorinating it in the presence of mercury fluoride, or, preferably, as described in detail in EP-A-0 522639, by fluorination of CCl₃-CH₂-CCl₃ with HF in the presence of a catalyst.

Thus, a preferred embodiment of the present invention concerns a process for the preparation of trifluoropropionic acid from CF₃-CH₂-CCl₂F and CF₃-CH₂-CClF₂ or a mixture thereof, wherein CF₃-CH₂-CCl₂F and CF₃-CH₂-CClF₂ or a mixture thereof have been prepared by fluorination of CCl₃-CH₂-CCl₃ with HF in the presence of a catalyst. The fluorination reaction can be performed especially as described in EP-A-0 522639. According to that publication, 1,1,1,3,3,3-hexachloropropane is reacted with HF in the presence of a catalyst, such as one or more derivatives of metals selected from the groups IIIa, IVa and IVb, Va and Vb, VIa and Vlb of the periodic system of the elements, especially derivatives of titanium, tantalum, molybdenum, boron, tin and antimony. Derivatives of antimony, tin and titanium are preferred. As derivatives, salts, especially the halides, especially preferably the chlorides, fluorides and chlorofluorides are to be mentioned.

The catalyst is generally applied in an amount of at least 0.005 moles per mole and at most 0.1 moles per mole of the hexachloropropane. The molar ratio of HF to the hexachloropropane generally lies in the range of 4:1 up to 20:1. The reaction temperature is generally at least 50 °C, but not higher than 150 °C. The pressure is preferably selected so that the reaction mixture remains in the liquid state and is dependent from the temperature of the reaction mixture. Usually, the pressure is at least 2 bar (abs.), but not higher than 50 Bar (abs.).

If desired the reaction with oleum may be performed in the presence of a catalyst, such as a Lewis acid.

The resulting fluorination products can be separated as usual, e.g. by distillation, to obtain the respective tri-, tetra- and pentafluorinated product. Of course, it is possible to use a mixture of two or all three fluorination products without separating them beforehand in the process of the present invention. This, of course, is an advantage.

1,1,1,3,3,3-hexachloropropane can be prepared in any manner which seems convenient. As already indicated in the cited EP-A-0 522639, 1,1,1,3,3,3-hexachloropropane can advantageously be prepared by the addition of tetrachloromethane to vinylidene chloride, like it is known from a publication of Belbachir et al. in Makromol. Chem. 185 (1984), pages 1583 to 1595. This reaction can be performed in the presence of peroxide catalysts or iron or copper salts, preferably in a polar solvent like dimethylsulfoxide or acetonitrile or with co-catalysts like amines. A preferred embodiment of the present invention concerns the preparation of trifluoropropionic acid starting by preparing 1,1,1,3,3,3-hexachloropropane by addition of tetrachloromethane to vinylidene chloride, fluorinating said hexachloropropane with HF to form CF₃-CH₂-CCl₃, CF₃-CH₂-CCl₂F and CF₃-CH₂-CClF₂ or a mixture thereof, especially to form CF₃-CH₂-CCl₂F and CF₃-CH₂-CClF₂ or a mixture thereof, and subsequent reaction with SO₃ and H₂SO₄ and hydrolysis.

The present invention provides for a process which yields the carboxylic acid in a simple manner; especially, trifluoropropionic acid can easily be produced. The invention also provides for improved embodiments to produce trifluoropropionic acid in a two steps or three steps process from fluorochloropropanes, which themselves are preferably produced from hexachloropropane, which in turn is preferably produced by the addition of tetrachloromethane to vinylidene chloride. Thus, trifluoropropionic acid can be produced from easily accessible starting materials in a technically simple process.

The following examples are intended to explain the invention in detail without the intention to limit its scope.

### Example 1: Preparation of hexachloropropane (HCPa) from vinylidene chloride (VC2) and tetrachloromethane (CCl₄) in the presence of CuCl₂ and tert-Butylamine (tBu)

The reaction was performed analogously to the process described in EP-A-0 522639. Details are compiled in table 1.

**Table 1**

| Molar ratios | | | | T [°C] | Duration [h] | Mass balance [%] | VC2 conversion [%] | Selectivity in HCPa | Yield [%] |
|---|---|---|---|---|---|---|---|---|---|
| VC2 | CCl4 | CuCl2 | tBu | | | | | | |
| 1 | 5 | 0.05 | 0.1 | 90 | 23 | 93 | 80 | 95 | 76 |

### Example 2: Fluorination of hexachloropropane with HF

The reaction between hexachloropropane and HF in the presence of antimony pentachloride as catalyst was performed analogously to the process as described in EP-A-0 522639. A mixture comprising HCFC-234fb, HCFC-235fa and HFC-236fa was formed. Details are compiled in table 2.

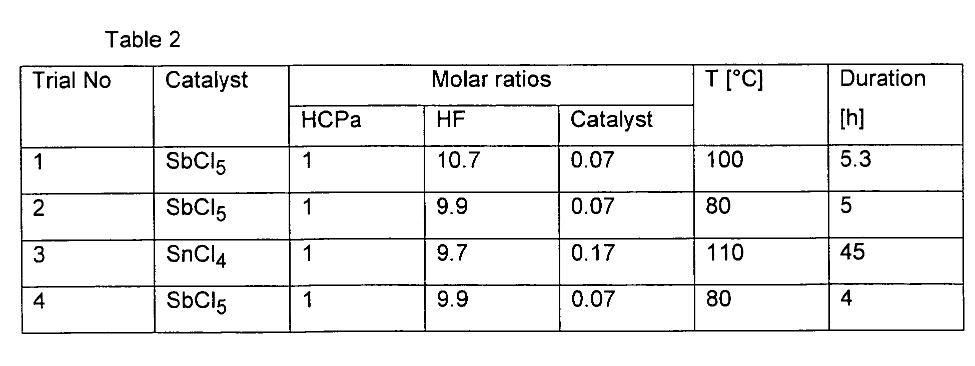

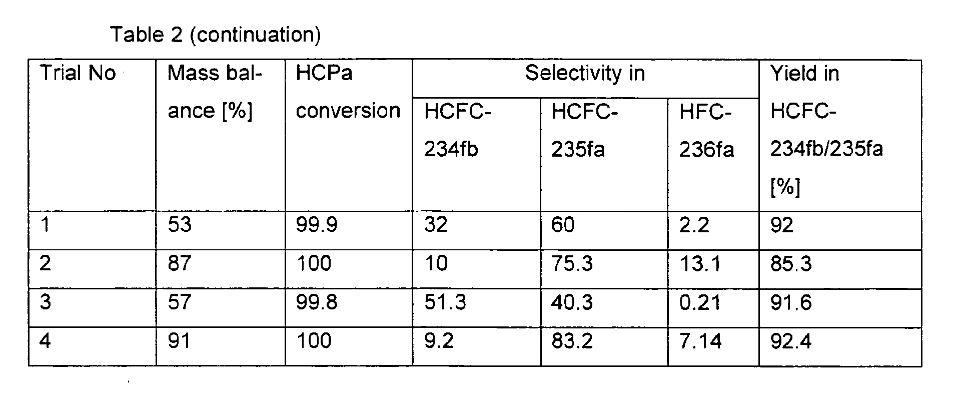

### Example 3: Hydrolysis of 1,1,1,3-tetrafluoro-3,3-dichloropropane and 1,1,1,3,3-pentafluoro-3-chloropropane mixtures

General procedure: The reactants 1,1,1,3-tetrafluoro-3,3-dichloropropane and 1,1,1,3,3-pentafluoro-3-chloropropane mixture and oleum were reacted in an autoclave under autogenous pressure under heating for a predetermined time. Then, water was added after cooling at room temperature and the trifluoropropionic acid formed was isolated by extraction with diethyl ether and distillation. Details for four trials are compiled in table 3.

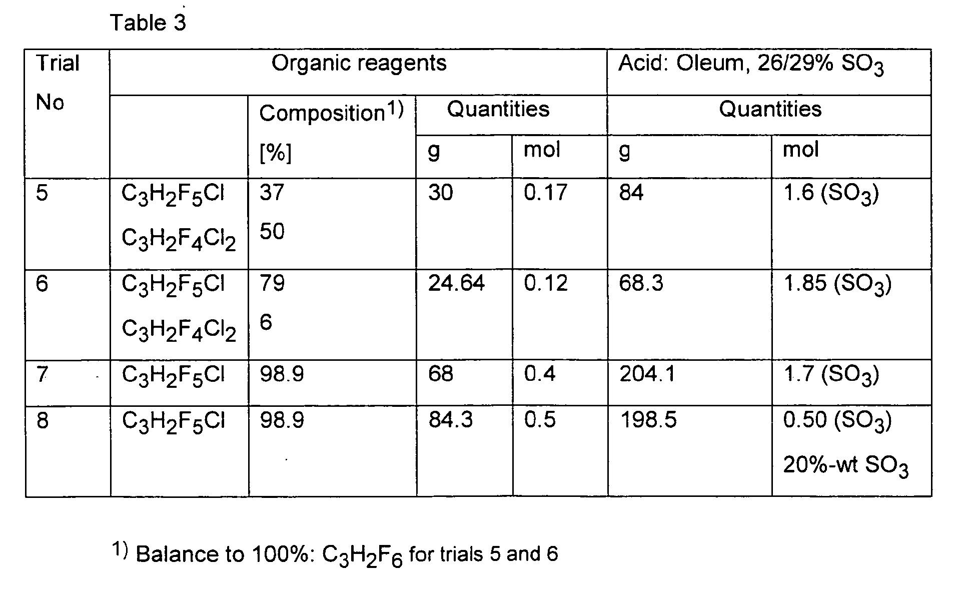

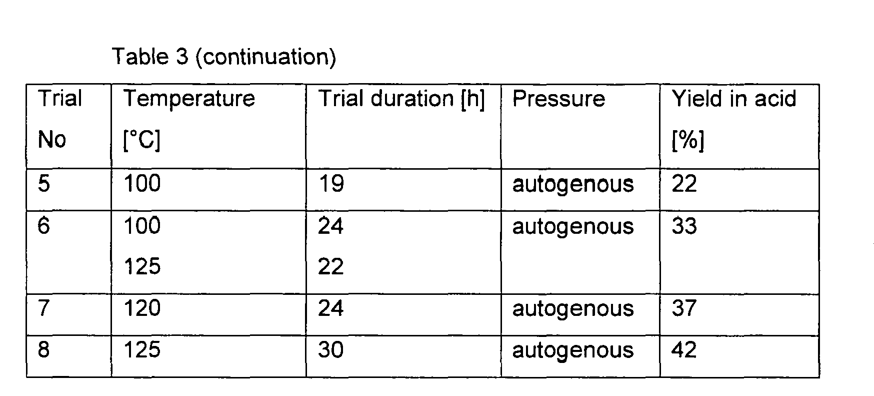

The examples 5 and 6 which are open for optimization demonstrate that trifluoropropionic acid can be obtained in fair yield in a technically very simple manner.

## Claims

1. Process for the preparation of carboxylic acids with the general formula (I) R-COOH wherein R is an alkyl group with 1 to 4 carbon atoms, optionally substituted by1 or more fluorine atoms, by reaction of a starting material of the general formula R-CCl₂F, R-CClF₂ or R-CCl₃ wherein R has the meaning given above with SO₃ and H2SO₄ and subsequent hydrolysis, with the exception of the preparation of trifluoroacetic acid from CF₃CCl₃.

2. Process according to claim 1 wherein R is an alkyl group with 2 to 4 carbon atoms.

3. Process according to claim 1 wherein R is a R'-CH₂ group wherein R' is an alkyl group with 1 to 3 carbon atoms which is substituted by at least 2 fluorine atoms.

4. Process according to claim 3 for the preparation of 3,3,3-trifluoropropionic acid by reacting CF₃-CH₂-CCl₂F, CF₃-CH₂-CClF₂, CF₃-CH₂-CCl₃ or a mixture thereof as starting material with sulfur trioxide.

5. Process according to claim 4 wherein the starting material comprises CF₃-CH₂-CCl₂F, CF₃-CH₂-CClF₂ or a mixture thereof.

6. Process according to claim 5, wherein the starting material is contacted with sulfur trioxide and sulfuric acid in the form of oleum.

7. Process according to claim 6, wherein the oleum (initially) comprises sulfur trioxide in an amount of 20 %-wt to 40 %-wt.

8. Process according to claim 1, **characterized by** a molar ratio between sulfur trioxide and starting material between 1:1 and 5:1.

9. Process according to claim 1, wherein the reaction is carried out at a temperature between 90 and 130°C.

10. Process according to claim 1, wherein the pressure lies between 1 and 30 bar (abs.).

11. Process according to claim 4, wherein the starting material is prepared by fluorination of CCl₃-CH₂-CCl₃.

12. Process according to claim 11, wherein CCl₃-CH₂-CCl₃ is fluorinated with HF in the presence of a catalyst.

13. Process according to claim 12 wherein CCl₃-CH₂-CCl₃ is prepared by the reaction of CH₂=CCl₂ and CCl₄.
